# EUROPEAN PATENT SPECIFICATION

(11) **EP 2 064 997 B1**
(45) Date of publication and mention of the grant of the patent: **27.04.2011**
(21) Application number: 09155111.9
(22) Date of filing: 30.05.2003
(51) Int. Cl.: A61B 10/00, A61B 17/34

(54) **Apparatus to access the bone marrow**
Vorrichtung für den Zugriff auf Knochenmark
Appareil d'accès à la moelle osseuse

(30) Priority: 31.05.2002 US 384756 P
(43) Date of publication of application: 03.06.2009
(62) Divisional of application: 03756317.8
(73) Proprietor: Vidacare Corporation, Shavano Park, TX 78249-2095 (US)
(72) Inventor: Miller, Larry J., Spring Branch, TX 78070-6127 (US)
(74) Representative: Lawrence, John

(56) References cited:
- EP-A- 0 807 412
- WO-A-93/07819
- WO-A-96/31164
- WO-A-98/06337
- FR-A- 853 349
- GB-A- 2 130 890

## Description

### TECHNICAL FIELD

The present invention is related to an apparatus for withdrawing specimens from the bone or bone marrow. The apparatus can be used to extract stem cells or bone marrow for transplantation or diagnostic purposes.

### BACKGROUND

There are many clinical conditions where it is important to be able to access and retrieve bone marrow. In some cases it may be necessary to treat diseases with a bone marrow or stem cell transplant to restore functioning blood cells in the body after high-dose chemotherapy. Such conditions may include acute leukemias, brain tumors, breast cancer, Hodgkin's disease, multiple myeloma, neuroblastoma, non-Hodgkin's lymphomas, ovarian cancer, sarcoma and testicular cancer. In other cases it is necessary to access the bone marrow to obtain a sample of the marrow for diagnostic testing. These conditions may include cancer of any type and hematologic disease of any origin.

Current techniques for gaining access to bone marrow can be difficult, traumatic and occasionally dangerous, depending on the site selected for harvest, the operator's expertise and the patient's anatomy. In general, the devices available for gaining access to the medullary cavity of the bone, where the bone marrow is located, include a trocar-fitted needle, with handles to facilitate application of pressure and rotation. These types of devices require substantial force to break through the outer cortex of the bone by a fracturing technique. The exertion of high pressure upon the needle causes pain for the patient and often damages the tip of the needle. This is particularly a problem when harvesting from the sternum of a patient because the excess force can cause penetration through the sternum and can damage underlying structures such as the heart and great vessels.

Another disadvantage of current techniques to access the bone marrow is that frequently more than one penetration site into the bone is required to retrieve enough bone marrow to either perform diagnostic tests or for transplantation purposes. To retrieve an adequate sample of bone marrow for either a bone marrow or stem cell transplant, a physician may need to put the needle into several different parts of the pelvis which may require up to six needle puncture sites or more. This multiple-pass requirement can be extremely painful for a patient and may deter people from donating bone marrow. This technique of using multiple passes can also cause fatigue in smaller operators who may lack strength to complete multiple-pass procedures.

Retrieving bone samples for diagnostic purposes is likewise difficult. Occasionally the core sample of bone is not retrieved because it is not extracted successfully with a standard biopsy needle. Thus, multiple attempts may be necessary to obtain a satisfactory bone or bone marrow biopsy.

Current techniques require that biopsy needles be forced by manual pressure into bone. These techniques may have undesired side effects such as damaging a needle tip or having the needle slide off the proposed bone target into organs or soft tissues.

The reader may be further enlightened as to the state of the art by reference to the closest prior art of FR 853349.

### SUMMARY OF THE INVENTION

There is a need for an apparatus to access the bone marrow that is minimally traumatic to the patient and that allows sufficient amount of bone narrow to be removed the first time the bone is penetrated. It is an object of the present invention to provide an apparatus for the removal of portions of bone marrow from a bone.

According to the invention, this object can be achieved by apparatus according to claim 1. Preferred embodiments are denied by the dependent claims.

### BRIEF DESCRIPTION OF THE DRAWINGS

A more complete and thorough understanding of the An apparatus for removing portions of bone and bone marrow from a bone and advantages thereof may be acquired, by way of example, by referring to the following description taken in conjunction with the accompanying drawings, in which like reference numbers indicate like features, and wherein:
FIGURE 1A illustrates an apparatus not falling under the scope of the invention for removing a bone marrow sample shown in longitudinal cross section.
FIGURE 1B illustrates a hollow drive shaft.
FIGURE 1C illustrates an embodiment of a trocar.
FIGURE 1D illustrates an embodiment of an apparatus.
FIGURE 1E illustrates an embodiment of an apparatus according to the invention for removing a bone marrow sample shown in longitudinal cross section.
FIGURE 2A illustrates an example of a hollow drive shaft or penetrator.
FIGURE 2B illustrates an example of a hollow drive shaft or penetrator.
FIGURE 2C illustrates an example of an inner trocar.
FIGURE 2D illustrates an example of a hollow drive shaft or penetrator.
FIGURE 3A illustrates an example of an attachment.
FIGURE 3B is an illustration of an embodiment of an attachment.
FIGURE 4A is an illustration of an embodiment of a hollow drive shaft and trocar.
FIGURE 4B is an illustration of an embodiment of a hollow drive shaft and trocar.
FIGURE 4C is an illustration of an embodiment of a hollow drive shaft and trocar in cross section.
FIGURE 4D is an illustration of an embodiment of a hollow drive shaft and trocar in cross section.
FIGURE 5A-C illustrates example gear assemblies.

### DETAILED DESCRIPTION OF THE APPARATUS

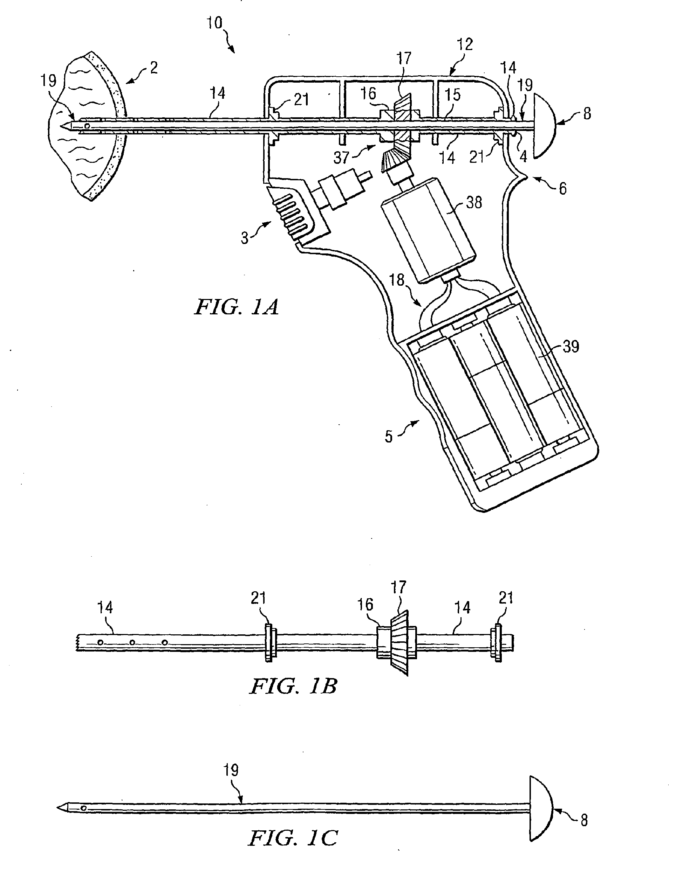

The construction, operation and advantages of the apparatus for taking a bone marrow sample are best understood by reference to FIGURES 1A-5C herein like numbers refer to same and like parts.

FIGURE 1A, illustrates an example of an apparatus for removing bone marrow from a bone. Apparatus 10 may be used to obtain a sample of bone marrow from any suitable bone in the body such as the iliac crest or the sternum. The apparatus 10 includes housing 12, a hollow drive shaft 14, removable trocar 19, gear assembly 37, motor 38 and power supply 39. Housing 12 may include on/off switch 3 handle 5 and guard 6. Handle 5 may be angled downward to allow ease of operation and also to allow end of hollow drive shaft 14 to turn without obstruction from a hand. Housing 12 encompasses power source 39, motor 38 and associated circuitry 18, hollow drive shaft 14 and gear assembly 37. Hollow drive shaft 14 includes inner channel 15 and collar 16. Gear 17 of gear assembly 37 engages collar 16 of hollow drill shaft 14 and thereby rotates hollow drive shaft 14.

Removable trocar 19 may be inserted into inner channel 15 of hollow drive shaft 14. Trocar 19 is hollow and has an inner channel operable to convey bone and bone marrow samples. Trocar 19 may have a handle 8 that can be used to tighten trocar 19 in place or to remove it from inner channel 15 of hollow drive shaft 14. Hollow drive shaft 14 may include luer lock connector 4 at the point where it exits housing 12. A luer lock connector may allow hollow drive shaft to connect to a suction apparatus such as a tubing or syringe or to any other suitable apparatus that would assist in obtaining a bone or bone marrow biopsy specimen. An access port, such as a suction port may also releasably attach to an end of hollow drive shaft where it exits housing 12, for example to a luer lock connector. Such an attachment may be a plug, a port, a suction apparatus, swivel port or other adapter.

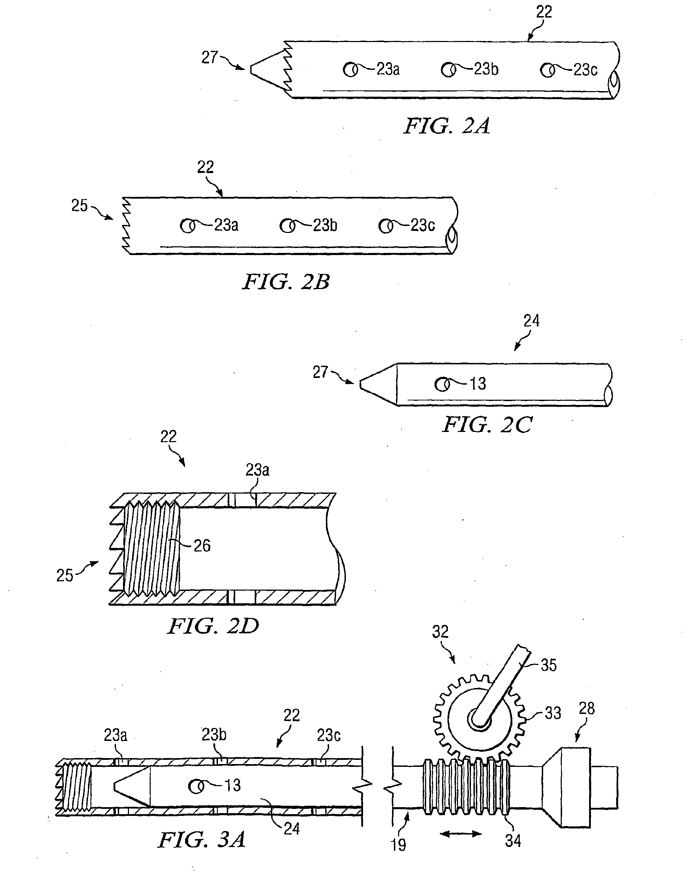

FIGURE 1B shows hollow drive shaft 14 removed from apparatus 10. In one embodiment, hollow drive shaft 14 may include one or more thrust bearings 21. Thrust bearings may absorb pressure from the thrust of a drive shaft into bone during drilling. Also included in hollow drive shaft are side ports shown in further detail in FIGURE 2. FIGURE 1C illustrates removable trocar 19 which includes handle 8. Handle 8 may be formed into a shape that is easily grasped and turned during the process of obtaining a biopsy. Removable trocar 19 may include one or more side ports operable to access a bone marrow specimen shown in greater detail in FIGURE 2.

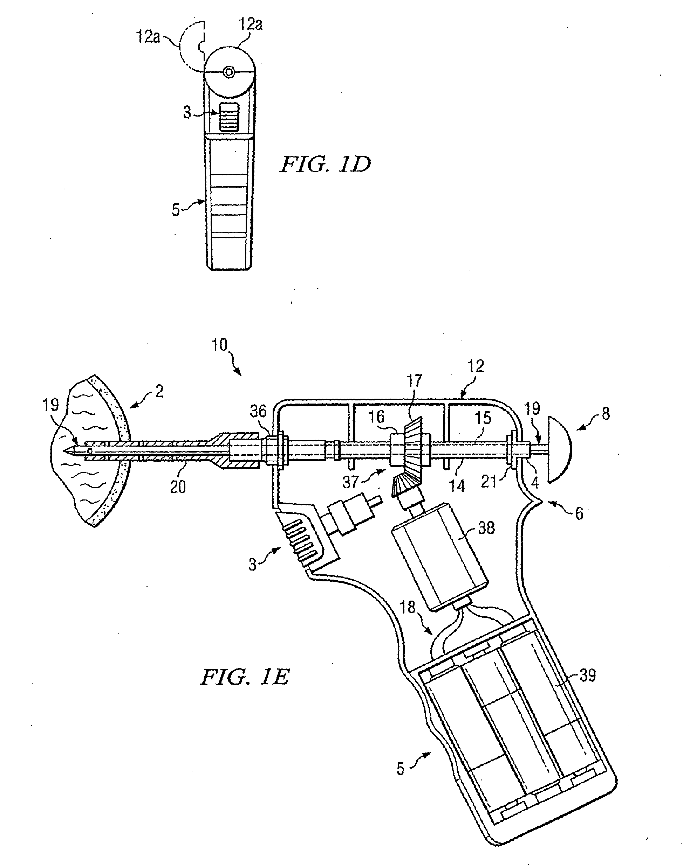

In one embodiment, shown in FIGURE 1D, housing 12 may include a releasable hatch or door 12a that may be opened to allow hollow drive shaft and attached gear 17 to be removed after use of apparatus 10. This may be desirable where apparatus 10 is reusable and hollow drill shaft 14 and removable trocar 19 are disposable. A releasable hatch or door may also be desirable to clean the inside of apparatus 10.

In an embodiment of the invention, shown in FIGURE 1E, detachable penetrator 20 may be attached to hollow drive shaft 14 by means of connector 36 and drilled into bone 2. In this embodiment, removable trocar 19 may be inserted into inner channel 15 of hollow drive shaft 14 and into the hollow channel of penetrator 20. Penetrator 20 may include side ports to permit access to bone and bone marrow samples during a biopsy or bone marrow harvest procedure. One advantage of an embodiment that includes a detachable penetrator is it allows penetrators of various sizes and configurations to be attached to hollow drive shaft 14. In this embodiment, connector 36 includes an inner channel operable to allow retrieval of bone and bone marrow specimens.

FIGURES 2A-D show an end 22 of either a hollow drive shaft or a penetrator that is suitable to penetrate a bone. FIGURE 2A, illustrates hollow drive shaft or penetrator end 22 which may include multiple sampling ports 23 through which bone marrow or other biopsy material may be aspirated. Sampling ports 23a, 23b and 23c are each operable to retrieve a portion of bone marrow. When removable trocar end 24 is in position within inner channel of hollow drive shaft end 22 sampling port 13 of removable trocar end 24 may align with sampling port 23a, 23b, or 23c. An operator may determine the level in the bone marrow where a specimen is taken or which sampling port (23a, 23b or 23c) to align with trocar sampling port 13. Hollow drive shaft or penetrator end 22 may include serrated tip 25 as shown in FIGURE 2B or any other suitable configuration for sampling bone or bone marrow.

FIGURE 2C shows removable trocar end 24 having a sampling port 23 near tip 27 through which a sample of bone or bone marrow can be retrieved as well as through a sampling port 23a, 23b or 23c. When sampling port 23a, 23b or 23c of hollow drive shaft or penetrator 22 is aligned with sampling port 13 of removable trocar 24, a portion of bone marrow may be suctioned out of the bone. Removable trocar 24 may be removed allowing bone marrow to be suctioned through one or more sampling ports 23 of hollow drive shaft or penetrator 22 at different sites in a bone marrow cavity sequentially. FIGURE 2D shows another example of tip 25 of hollow drive shaft or penetrator end 22 having internal threads 26 that are able to engage a specimen of bone and core it out as hollow drive shaft or penetrator 22 is drilled into bone. Threads 26 may engage and adhere to a portion of bone or to a semisolid substance such as bone marrow and maintain contact with the specimen so it may be successfully retrieved.

FIGURE 3A shows operating mechanism 32 that may be included in some embodiments of the invention and may be attached to hollow drive shaft 14 and manipulated to change the depth in a bone marrow where sampling occurs. Operating mechanism 32 includes handle 35 and gear 33. Gear 33 engages gear 34 attached to trocar 19. Suction port 28, shown attached to trocar 19, may be used to retrieve portions of bone marrow from inner channel 15 of hollow drive shaft 14.

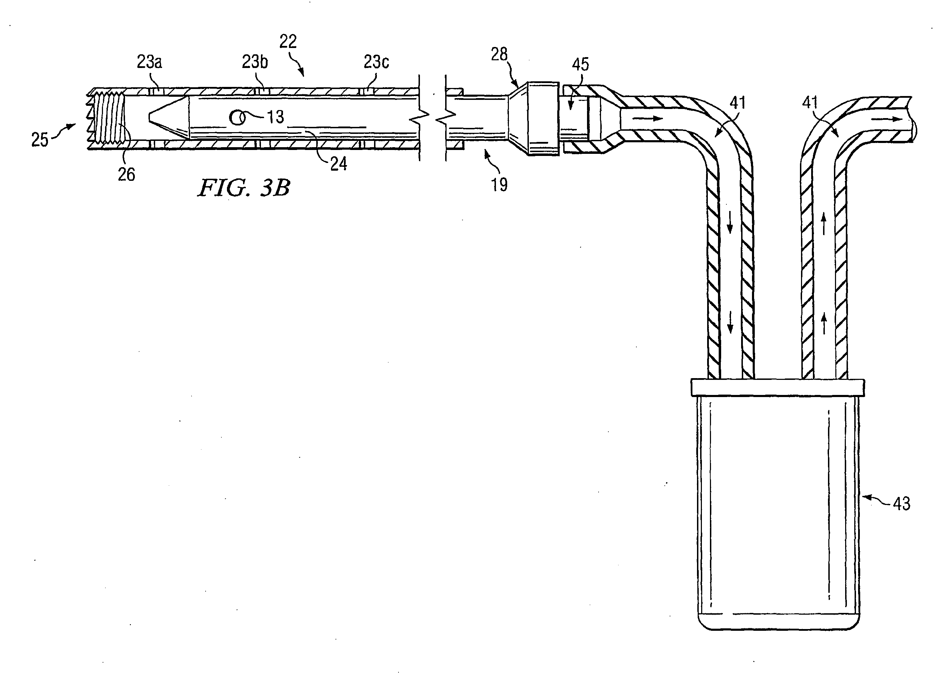

FIGURE 3B shows an example suction port 28 that may be connected to trocar 19 where it exits housing 12. In one embodiment a suction port or suction swivel apparatus may also be connected to the hollow drive shaft 14. This feature allows a suction apparatus of the type well known to one skilled in the art to be used to obtain bone marrow samples. Suction port 28 may also be configured to attach directly to a penetrator for example in an embodiment where the penetrator is detached from the housing before it is accessed for bone marrow retrieval. Also shown in FIGURE 3B is a suction swivel apparatus 45 that allows a suction tube 41 to attach to suction port 28. Suction swivel apparatus 45 allows suction port 28 to remain attached to suction tube 41 while an attached drilling apparatus is drilling into bone marrow without kinking or twisting suction tube 41. Also shown in FIGURE 3B is receptacle 43 that is interposed between suction tube 41 and a source of suction such that a bone marrow specimen may be successfully retrieved into receptacle 43 and not lost into a suction machine.

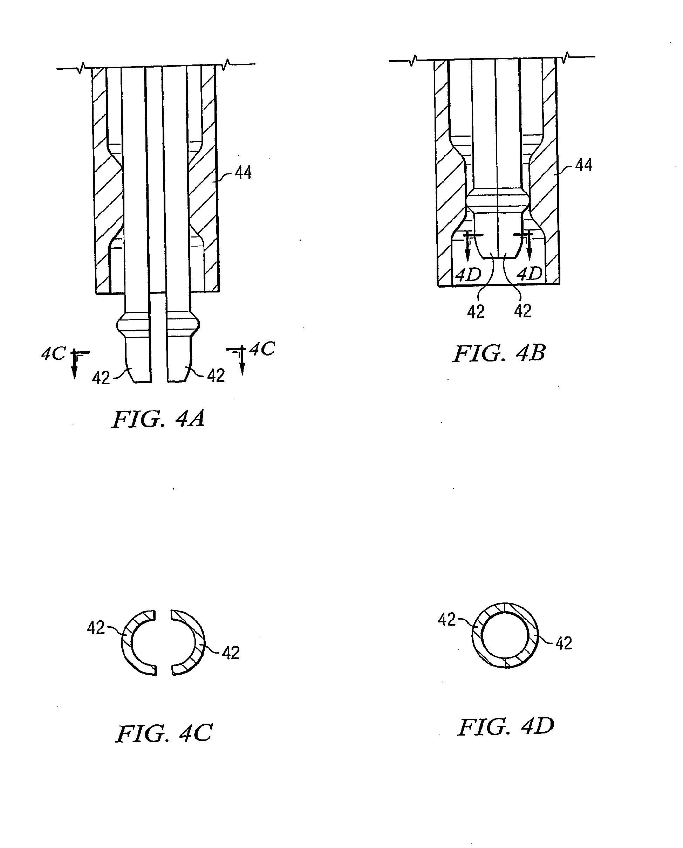

FIGURE 4A-D shows an example hollow drive shaft end or penetrator end 44 and inner trocar 42 having a split-needle configuration. An inner trocar 42 is inserted into a hollow drive shaft or penetrator end 44. Inner trocar 42 shown in the open position in FIGURE 4A is advanced past the end of hollow drive shaft end or penetrator end 44 where it may be opened and inserted into bone, bone marrow or other tissue. Inner trocar 42 may be closed, shown in FIGURE 4B, so that a specimen is retained in its grasp as it is withdrawn from the body. FIGURE 4C shows a cross section of the split-needle inner trocar 42 where the tip of the inner trocar 42 is in the open position. FIGURE 4D shows a cross section of the trocar 42 is shown in the closed position.

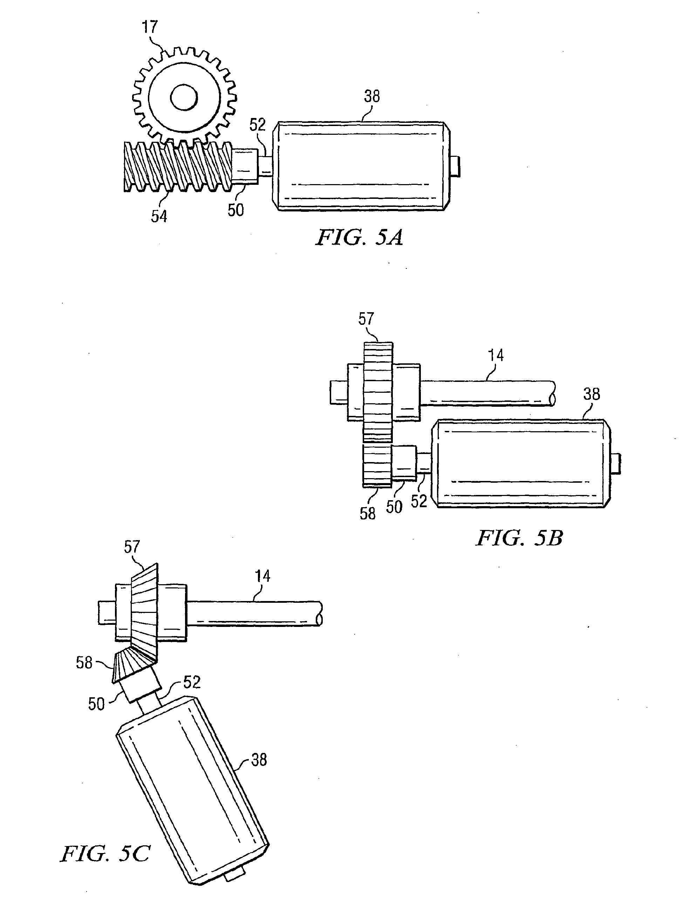

The apparatus may or may not include a reduction gear assembly. A reduction gear assembly may include a worm gear assembly shown in more detail in FIGURE 5A and may include first connector 50 that connects shaft 52 of motor 38 to worm gear 54. A reduction gear assembly may be used to decrease the RPMs between the motor and penetrator assembly to provide an optimum RPM at the point of insertion of penetrator assembly into bone. FIGURE 5B illustrates a further embodiment of a reduction gear assembly wherein a first spur gear 57 engages a second spur gear 58. FIGURE 5C illustrates an alternate embodiment of a reduction gear assembly wherein spur gear 56 is offset forty-five degrees relative to hollow drive shaft 14 which may be preferable in some embodiments. In this embodiment spur gear 58 may be offset at any angle and is not limited to forty-five degrees. Other gears may be used in a reduction gear assembly, for example a planetary gear (not expressly shown) may be used alone or in combination with a worm gear or a spur gear.

Although the present invention and its advantages have been described in detail, it should be understood that various changes, substitutions and alternations can be made herein without departing from the scope of the invention as defined by the following claims.

## Claims

1. An apparatus for removing portions of bone and bone marrow from a bone for diagnostic or therapeutic purposes comprising:
a housing (12);
a hollow drive shaft (14) disposed in the housing (12) and operable to engage a gear assembly (37) ;
the gear assembly (37) operable to rotate the hollow drive shaft (14); a penetrator (20) operable to penetrate the bone marrow and collect specimens of the bone marrow
a motor (38) coupled to the hollow drive shaft and operable to drive said penetrator (20) into the bone marrow by rotation of the hollow drive shaft;
a power supply (39) and associated circuitry operable to power the motor (38); an inner trocar (19) extending through an inner channel (15) of the drive shaft (14) and a passage through the penetrator (20) whereby a first end of said trocar is operable to penetrate the bone marrow and having a second end, the inner trocar (19) is removable from the inner channel (15) and the passage and a connector (36) operable to releasably attach said penetrator (20) to the distal end of the hollow drive shaft
the motor being and in that the hollow drive shaft (14) is **characterised in that** the hollow drive shaft (14) comprises an inner channel (15) operable to convey portions of bone marrow to an operator, and a proximal end operable to allow retrieval of portions of bone marrow.

2. The apparatus of Claim 1 wherein the inner trocar (19) comprises a tip (27) having a longitudinal groove operable to release bone chips from an insertion site into the bone marrow.

3. The apparatus of Claim 1 or 2 wherein the second end of the hollow drive shaft (14) comprises an access port operable to be used to obtain portions of bone and bone marrow from the inner channel (15).

4. The apparatus according to any one of the preceding claims wherein the second end of the hollow drive shaft (14) comprises a suction port (28) to remove portions of bone and bone marrow into a receptacle.

5. The apparatus according to any one of the preceding claims wherein the second end of the inner trocar (19) comprises a ratcheted gear operable to control the depth of sampling from the bone marrow.

6. The apparatus according to any one of the preceding claims wherein the penetrator (20) comprises internal threads operable to engage bone or bone marrow as the penetrator (20) is drilled into the bone.

7. The apparatus according to any one of the preceding claims wherein the inner trocar (19) comprises two separate longitudinal bodies that can move together to grasp a biopsy specimen.

8. The apparatus according to any one of the preceding claims wherein the gear assembly (37) comprises a reduction gear assembly.

9. The apparatus according to any one of the preceding claims wherein the hollow drive shaft (14) comprises at least one thrust bearing.

10. The apparatus according to any one of the preceding claims wherein the penetrator (20) comprises at least one side port.

11. The apparatus according to any one of the preceding claims wherein the removable inner trocar (19) comprises at least one side port operable to align with a side port of the penetrator (20).

12. The apparatus according to any one of the preceding claims wherein the hollow drive shaft (14) comprises a luer lock connector (4).

13. The apparatus of claim 1 comprising a housing (12) including a releasable hatch or door (12a) whereby the hollow drive shaft (14) can be installed and removed.

## Patentansprüche

1. Eine Vorrichtung zur Entfernung von Teilen von Knochen und Knochenmark von einem Knochen für diagnostische oder therapeutische Zwecke umfassend:
ein Gehäuse (12);
eine hohle Antriebswelle (14), die in dem Gehäuse (12) angeordnet ist und geeignet ist, eine Getriebeanordnung (37) in Eingriff zu nehmen;
die Getriebeanordnung (37) ist geeignet, die hohle Antriebswelle (14) zu rotieren;
einen Eindringkörper (20), der geeignet ist, in das Knochenmark einzudringen und Proben des Knochenmarks zu sammeln;
einen Motor (38), der an die hohle Antriebswelle gekoppelt ist und der geeignet ist, diesen Eindringkörper (20) in das Knochenmark durch Rotation der hohlen Antriebsachse zu führen;
eine Energieversorgung (39) und eine dazugehörige Schaltung, die geeignet ist den Motor (38) anzuschalten;
einen inneren Trokar (19), der sich durch einen inneren Kanal (15) der Antriebswelle (14) und einen Durchgang des Eindringkörpers (20) erstreckt, wobei ein erstes Ende dieses Trokars geeignet ist, in das Knochenmark einzudringen und der ein zweites Ende aufweist,
der innere Trokar (19) ist aus dem inneren Kanal (15) und dem Durchgang entfernbar und
ein Anschlussteil (36), das geeignet ist, diesen Eindringkörper (20) lösbar an dem distalen Ende der hohlen Antriebswelle zu befestigen;
**dadurch gekennzeichnet, dass** die hohle Antriebswelle (14) einen inneren Kanal (15), der geeignet ist, Teile des Knochenmarks zu einem Bediener zu befördern, und ein proximales Ende, das geeignet ist, eine Bereitstellung von Teilen des Knochenmarks zu erlauben, umfasst.

2. Die Vorrichtung nach Anspruch 1, wobei der innere Trokar (19) eine Spitze (27) umfasst, die einen längslaufenden Einschnitt aufweist, der geeignet ist, Knochenspähne aus einer Eindringstelle in dem Knochenmark freizugeben.

3. Eine Vorrichtung nach Anspruch 1 oder 2, wobei das zweite Ende der hohlen Antriebswelle (14) einen Zugangsanschluss umfasst, der geeignet ist, um verwendet zu werden, um Teile des Knochen und des Knochenmarks von dem inneren Kanal (15) zu erhalten.

4. Die Vorrichtung nach einem der vorhergehenden Ansprüche, wobei das zweite Ende der hohlen Antriebswelle (14) einen Absauganschluss (28) umfasst, um Teile des Knochens und des Knochenmarks in ein Gefäß zu entfernen.

5. Die Vorrichtung nach einen der vorhergehenden Ansprüche, wobei das zweite Ende des inneren Trokars (19) eine Sperreinrichtung umfasst, die geeignet ist, die Tiefe der Probennahme von dem Knochenmark zu kontrollieren.

6. Die Vorrichtung nach einem der vorhergehenden Ansprüche, wobei der Eindringkörper (20) innere Gewinde umfasst, die geeignet sind, Knochen oder Knochenmark aufzunehmen, wenn der Eindringkörper (20) in den Knochen gedreht wird.

7. Die Vorrichtung nach einem der vorhergehenden Ansprüche, wobei der innere Trokar (19) zwei separate längslaufende Körper umfasst, die sich zusammen bewegen können, um eine Biopsieprobe zu fassen.

8. Die Vorrichtung nach einem der vorhergehenden Ansprüche, wobei die Getriebeanordnung (37) eine Reduziergetriebeanordnung umfasst.

9. Die Vorrichtung nach einem der vorhergehenden Ansprüche, wobei die hohle Antriebswelle (14) mindestens ein Axiallager umfasst.

10. Die Vorrichtung nach einem der vorhergehenden Ansprüche, wobei der Eindringkörper (20) mindestens einen Seitenanschluss umfasst.

11. Die Vorrichtung nach einen der vorhergehenden Ansprüche, wobei der entfernbare innere Trokar (19) mindestens einen Seitenanschluss umfasst, der geeignet ist, um sich mit einem Seitenanschluss des Eindringkörpers (20) auszurichten.

12. Die Vorrichtung nach einem der vorhergehenden Ansprüche, wobei die hohle Antriebswelle (14) ein Luer-Lock Anschlussteil (4) umfasst.

13. Die Vorrichtung nach Anspruch 1, umfassend ein Gehäuse (12), das eine entfernbare Klappe oder Tür (12a) umfasst, wodurch die hohle Antriebswelle (14) installiert oder entfernt werden kann.

## Revendications

1. Appareil pour retirer des parties d'os et de moelle osseuse d'un os pour des buts de diagnostic et thérapeutiques, comprenant :
un boîtier (12) ;
une tige d'entraînement creuse (14) disposée dans le boîtier (12) et pouvant fonctionner pour mettre en prise un ensemble d'engrenages (37) ;
l'ensemble d'engrenage (37) étant opérationnel pour faire tourner la tige d'entraînement creuse (14) ;
un dispositif de pénétration (20) opérationnel pour pénétrer dans la moelle osseuse et collecter des échantillons de la moelle osseuse ;
un moteur (38) couplé à la tige d'entraînement creuse et pouvant fonctionner pour entraîner ledit dispositif de pénétration (20) dans la moelle osseuse grâce à la rotation de la tige d'entraînement creuse ;
une alimentation de puissance (39) et des circuits associés pouvant fonctionner pour alimenter le moteur (38) ; un trocart interne (19) s'étendant à travers un canal interne (15) de la tige d'entraînement (14) et un passage à travers le dispositif de pénétration (20), moyennant quoi une première extrémité dudit trocart peut fonctionner pour pénétrer dans la moelle osseuse et ayant une deuxième extrémité, le trocart interne (19) peut être retiré du canal interne (15) et du passage, et un connecteur (36) pouvant fonctionner pour fixer de manière amovible ledit dispositif de pénétration (20) sur l'extrémité distale de la tige d'entraînement creuse ;
et en ce que la tige d'entraînement creuse (14) est **caractérisé en ce que** la tige d'entraînement creuse (14) comprend un canal interne (15) pouvant fonctionner pour transporter des parties de la moelle osseuse jusqu'à un opérateur et une extrémité proximale pouvant fonctionner pour permettre la récupération des parties de moelle osseuse.

2. Appareil selon la revendication 1, dans lequel le trocart interne (19) comprend une pointe (27) ayant une rainure longitudinale pouvant fonctionner pour libérer des copeaux d'os d'un site d'insertion dans la moelle osseuse.

3. Appareil selon la revendication 1 ou 2, dans lequel la deuxième extrémité de la tige d'entraînement creuse (14) comprend un orifice d'accès opérationnel pour être utilisé afin d'obtenir des parties d'os et de moelle osseuse du canal interne (15).

4. Appareil selon l'une quelconque des revendications précédentes, dans lequel la deuxième extrémité de la tige d'entraînement creuse (14) comprend un orifice d'aspiration (28) pour retirer des parties d'os et de moelle osseuse dans un réceptacle.

5. Appareil selon l'une quelconque des revendications précédentes, dans lequel la deuxième extrémité du trocart interne (19) comprend un engrenage encliqueté pouvant fonctionner pour contrôler la profondeur d'échantillonnage de la moelle osseuse.

6. Appareil selon l'une quelconque des revendications précédentes, dans lequel le dispositif de pénétration (20) comprend des filetages internes opérationnels pour mettre en prise l'os ou la moelle osseuse au fur et à mesure que le dispositif de pénétration (20) perce l'os.

7. Appareil selon l'une quelconque des revendications précédentes, dans lequel le trocart interne (19) comprend deux corps longitudinaux séparés qui peuvent se déplacer ensemble pour saisir un échantillon de biopsie.

8. Appareil selon l'une quelconque des revendications précédentes, dans lequel l'ensemble d'engrenage (37) comprend un ensemble d'engrenage de réduction.

9. Appareil selon l'une quelconque des revendications précédentes, dans lequel la tige d'entraînement creuse (14) comprend au moins un palier de butée.

10. Appareil selon l'une quelconque des revendications précédentes, dans lequel le dispositif de pénétration (20) comprend au moins un orifice latéral.

11. Appareil selon l'une quelconque des revendications précédentes, dans lequel le trocart interne amovible (19) comprend au moins un orifice latéral opérationnel pour s'aligner avec un orifice latéral du dispositif de pénétration (20).

12. Appareil selon l'une quelconque des revendications précédentes, dans lequel la tige d'entraînement creuse (14) comprend un connecteur Luer (4).

13. Appareil selon la revendication 1, comprenant un boîtier (12) comprenant une trappe ou porte amovible (12a) moyennant quoi la tige d'entraînement creuse (14) peut être installée et retirée.
